(19) Europäisches Patentamt European Patent Office Office européen des brevets

(11) **EP 3 154 776 B1**

(12) **EUROPEAN PATENT SPECIFICATION**

(45) Date of publication and mention of the grant of the patent:
**19.01.2022 Bulletin 2022/03**

(21) Application number: **15731214.1**

(22) Date of filing: **12.06.2015**

(51) International Patent Classification (IPC):
**B32B 5/02** *(2006.01)*    **B32B 27/08** *(2006.01)*
**B32B 27/12** *(2006.01)*    **B32B 27/20** *(2006.01)*
**B32B 27/32** *(2006.01)*    **B32B 3/26** *(2006.01)*
**A61F 13/15** *(2006.01)*    **A61F 13/514** *(2006.01)*
**B32B 7/04** *(2019.01)*

(52) Cooperative Patent Classification (CPC):
**A61F 13/51462; A61F 13/15; A61F 13/5148;**
**B32B 5/022; B32B 7/04; B32B 27/08; B32B 27/12;**
**B32B 27/205; B32B 27/32;** B32B 3/26;
B32B 2250/246; B32B 2264/102; B32B 2264/104;
B32B 2270/00; B32B 2307/102;    (Cont.)

(86) International application number:
**PCT/US2015/035454**

(87) International publication number:
**WO 2015/191942 (17.12.2015 Gazette 2015/50)**

(54) **MULTILAYER FILMS, AND ARTICLES MADE THEREFROM**

MEHRSCHICHTFOLIEN UND DARAUS HERGESTELLTE ARTIKEL

FILMS MULTICOUCHES ET ARTICLES FABRIQUÉS À PARTIR DE CEUX-CI

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.06.2014 US 201462011227 P**

(43) Date of publication of application:
**19.04.2017 Bulletin 2017/16**

(73) Proprietors:
  • **Dow Global Technologies LLC**
    **Midland, MI 48674 (US)**
  • **Dow Quimica Mexicana S.A.de C.V.**
    **11000 Mexico Distrito Federal (MX)**

(72) Inventors:
  • **ARTEAGA LARIOS, Fabricio**
    **06500 Mexico City (MX)**
  • **DE GROOT, Jackie**
    **Freeport, Texas 77541 (US)**
  • **SHAH, Viraj K.**
    **Freeport, Texas 77541 (US)**
  • **KALIHARI, Vivek**
    **Freeport, Texas 77541 (US)**

(74) Representative: **Boult Wade Tennant LLP**
    **Salisbury Square House**
    **8 Salisbury Square**
    **London EC4Y 8AP (GB)**

(56) References cited:
| | |
|---|---|
| WO-A1-00/06381 | WO-A1-00/38915 |
| WO-A1-01/83599 | WO-A1-98/02610 |
| WO-A1-98/58799 | WO-A1-99/14047 |
| WO-A1-99/23139 | WO-A1-03/020513 |
| WO-A2-2004/024433 | US-A1- 2003 106 560 |
| US-A1- 2004 122 388 | US-A1- 2005 118 435 |

**(Cont. next page)**

(52) Cooperative Patent Classification (CPC): (Cont.)
B32B 2307/514; B32B 2307/558; B32B 2307/72;
B32B 2307/724; B32B 2535/00; B32B 2555/00;
B32B 2555/02

## Description

### FIELD

Embodiments of the present disclosure generally relate to multilayer films and applications of the multilayer films to make articles, such as, for example, ultrasonically-bonded laminates.

### BACKGROUND

Cloth-like backsheets have become increasingly desirable for use in hygiene absorbent products, such as, for example, diapers, adult incontinence products, and feminine hygiene articles, in order to provide good haptics, such as softness, and low noise, while still offering sufficient barrier properties to perform its primary function of containing fluids. Cloth-like backsheets typically include a nonwoven substrate and a film laminated together, and depending on the lamination technology involved, the haptics of the backsheet can vary. Several different lamination technologies exist for joining films and nonwovens, and can include, for example, extrusion coating, hot melt adhesive, solvent-less adhesives, and ultrasonic bonding. Each lamination technique has its own particularities. In recent years, ultrasonic bonding has become an emerging lamination technology for use in producing backsheets; however, it is not without its challenges. One major challenge observed when using ultrasonic bonding is that where different types of materials are used for the nonwoven substrate and the film, (e.g., a polyethylene-based film laminated to a polypropylene nonwoven substrate), adhesion is adversely affected often resulting in a poor bond between the two. In addition, pinholes can result which can destroy the liquid barrier functionality of the backsheet.

US 2003/106560 A1 relates to a nonwoven filled film laminate with barrier properties.

Accordingly, alternative multilayer films that can provide good adhesion to a nonwoven polypropylene substrate, and articles comprising multilayer films having good haptics, such as softness, and low noise, as well as, reduced pinholes are desired.

### SUMMARY

The invention is in accordance with the appended claims.

Disclosed in embodiments herein are multilayer films. The films as claimed comprise a core layer and two skin layers, wherein the core layer is positioned between the two skin layers, wherein the core layer comprises a polyethylene polymer blend, the polyethylene polymer blend comprising at least 40%, by weight of the polyethylene polymer blend, of an ethylene-based polymer having a density of 0.900-0.935 g/cc and a melt index of 0.7-6 g/10 min, wherein the polyethylene polymer blend has an overall density of about 0.910-0.945 g/cc and a melt index of about 0.7-6 g/ 10 min, and wherein each skin layer independently comprises a propylene-based polymer; wherein the propylene-based polymer:

(a) is a propylene homopolymer; or
(b) comprises a polypropylene polymer blend, wherein the polypropylene polymer blend comprises from 10 wt% to 30 wt% of a low density polyethylene and greater than 50 wt.% of a propylene-based polymer, by weight of the polypropylene polymer blend;

and where the skin layer is not a non-woven material. In embodiments herein, the multilayer films may be polyethylene-based.

In some embodiments herein, the polyethylene polymer blend further comprises a low density polyethylene having a density of about 0.915-0.930 g/cc and a melt index of about 1-15 g/10 min. In some embodiments herein, the polyethylene polymer blend comprises less than 30%, by weight of the polyethylene polymer blend, of the low density polyethylene. In some embodiments herein, the polyethylene polymer blend further comprises a medium or high density polyethylene having a density of about 0.930-0.965 g/cc and a melt index of about 1-10 g/10 min. In some embodiments herein, the polyethylene polymer blend comprises 15% to 30%, by weight of the polyethylene polymer blend, of the medium or high density polyethylene. In some embodiments herein, the polyethylene polymer blend further comprises 5% to 15%, by weight of the polyethylene polymer blend, of a low density polyethylene having a density of about 0.915-0.930 g/cc and a melt index of about 1-15 g/10 min, and 15% to 25%, by weight of the polyethylene polymer blend, of a medium or high density polyethylene having a density of about 0.930-0.965 g/cc and a melt index of about 1-10 g/10 min.

In some embodiments herein, the propylene-based polymer comprises a polypropylene polymer blend that further comprises a low density polyethylene having a density of about 0.915-0.930 g/cc and a melt index of about 1-15 g/10 min.

In some embodiments herein, the core layer comprises from about 50% to about 80% of the overall film thickness. In some embodiments herein, the two skin layers have an equal thickness. In some embodiments, the two skin layers may not have equal thicknesses. In some embodiments herein, each skin layer further comprises a compatibilizer agent capable of compatibilizing blends of polyethylene and polypropylene polymers. In some embodiments herein, the compatibilizer agent comprises polyolefin plastomers or polyolefin elastomers.

In some embodiments herein, the film has a basis weight of between about 10-20 gsm. In some embodiments herein, the film exhibits at least one of the following properties: a spencer dart impact strength of greater than 140 g, a 2% secant modulus of greater than about 110316 kPa (16,000 psi) in the MD and greater than 110316 kPa (16,000 psi) in the CD, a stress at break in the cross-direction of greater than about 11721 kPa (1,700 psi), and in the machine direction of greater than about 13790 kPa (2,000 psi), or a puncture resistance greater than about 1.24 J/cm$^3$ (15 ft·lb$_f$/in$^3$). In some embodiments herein, the film exhibits at least one of the following properties: a softness value difference of less than 5%, when compared to a 100% polyethylene film having a 2% secant modulus greater than about 110316 kPa (16,000 psi) in the machine direction; or a noise value of less than 0.5 dB between a frequency band of 1,000 Hz and 5,000 Hz.

Also disclosed in embodiments herein are ultrasonically bonded laminates. The laminates comprise a multilayer film according to one or more embodiments herein, and a nonwoven substrate at least partially ultrasonically bonded to the multilayer film. In some embodiments herein, the nonwoven substrate is made from a propylene-based material. In some embodiments herein, the laminate exhibits at least one of the following properties: a peel force value of greater than about 1.2 N, or a hydrostatic pressure above 7 kPa (70 mbar).

Additional features and advantages of the embodiments will be set forth in the detailed description which follows, and in part will be readily apparent to those skilled in the art from that description or recognized by practicing the embodiments described herein, including the detailed description which follows, the claims, as well as the appended drawings.

It is to be understood that both the foregoing and the following description describe various embodiments and are intended to provide an overview or framework for understanding the nature and character of the claimed subject matter. The accompanying drawings are included to provide a further understanding of the various embodiments, and are incorporated into and constitute a part of this specification. The drawings illustrate the various embodiments described herein, and together with the description serve to explain the principles and operations of the claimed subject matter.

## BRIEF DESCRIPTION OF THE DRAWINGS

FIG. 1 graphically depicts the 2% secant modulus for a multilayer film according to one or more embodiments shown or described herein in comparison to several comparative films.

FIG. 2 graphically depicts the load at break (i.e., stress at break) for a multilayer film according to one or more embodiments shown or described herein in comparison to several comparative films.

FIG. 3 graphically depicts the puncture resistance and spencer dart impact for a multilayer film according to one or more embodiments shown or described herein in comparison to several comparative films.

FIG. 4 graphically depicts the noise intensity for a multilayer film according to one or more embodiments shown or described herein in comparison to several comparative films.

FIG. 5 graphically depicts the softness for a multilayer film according to one or more embodiments shown or described herein in comparison to several comparative films.

## DETAILED DESCRIPTION

Reference will now be made in detail to embodiments of multilayer films and ultrasonically-bonded laminates, examples of which are further described in the accompanying figures. The multilayer films may be used to produce cloth-like backsheets. It is noted, however, that this is merely an illustrative implementation of the embodiments disclosed herein. The embodiments are applicable to other technologies that are susceptible to similar problems as those discussed above. For example, multilayer films used to produce cloth-like wipes, face masks, surgical gowns, tissues, bandages and wound dressings are clearly within the purview of the present embodiments. As used herein, "multilayer film" refers to a film having two or more layers that are at least partially contiguous and preferably, but optionally, coextensive.

In embodiments herein, the multilayer films comprise a core layer and two skin layers. The skin layers do not contain any non-woven materials. The core layer is positioned between the two skin layers. In an embodiment, the core layer may comprise more than two layers or more than three layers or more than five layers.

In some embodiments, the multilayer films may comprise one or more additional layers, such as structural, barrier, or tie layers, positioned between the core layer and each skin layer. Various materials can be used for these layers and can include polypropylenebased plastomers or elastomers, ethylene/vinyl alcohol (EVOH) copolymers, polyvinylidene chloride (PVDC), polyethylene terepthalate (PET), oriented polypropylene (OPP), ethylene/vinyl acetate (EVA) copolymers, ethylene/acrylic acid (EAA) copolymers, ethylene/methacrylic acid (EMAA) copolymers, polyacrylic imides, butyl acrylates, peroxides (such as peroxypolymers, e.g., peroxyolefins), silanes (e.g., epoxysilanes), reactive polystyrenes, chlorinated polyethylene, olefin block copolymers, propylene copolymers, propylene-ethylene copolymers, ULDPE, LLDPE, HDPE, MDPE, LMDPE, LDPE, ionomers, and graft-modified polymers (e.g., maleic anhydride grafted polyethylene).

The core layer comprises a polyethylene polymer blend, the polyethylene polymer blend comprising an ethylene-based polymer. Each skin layer independently comprises a propylene-based polymer. The propylene-based polymer may be a propylene homopolymer, or a polypropylene polymer blend.

In embodiments herein, the multilayer films may be polyethylene-based. As used herein in reference to multilayer films, "polyethylene-based" means that the multilayer films are primarily (i.e., greater than 50%, by total weight of the multilayer film) comprised of polyethylene resin. "Polyethylene" refers to a homopolymer of ethylene or a copolymer of ethylene with one or more comonomers with a majority of its polymer units derived from ethylene. Also disclosed herein are ultrasonically-bonded laminates comprising the multilayer films.

The thickness ratio of both skin layers to the core layer can be a ratio suitable to impart good ultrasonic bonding properties to the film. In some embodiments, the thickness ratio of both skin layers to the core layer may be 1:10 to 1:1. In other embodiments, the thickness ratio of both skin layers to the core layer may be 1:5 to 1:1. In further embodiments, the thickness ratio of both skin layers to the core layer may be 1:4 to 1:2. The thickness ratio of both skin layers to the core layer can also be captured by percentages. For example, in some embodiments, the core layer comprises greater than 50% to 90% of the overall film thickness. In other embodiments, the core layer comprises from 60% to 85% of the overall film thickness. In further embodiments, the core layer comprises from 65% to 80% of the overall film thickness. In embodiments herein, the two skin layers may have an equal thickness, or alternatively, may have an unequal thickness.

Core Layer

The core layer comprises a polyethylene polymer blend. As used herein, "polyethylene polymer blend" refers to a mixture of two or more polyethylene polymers. The polyethylene polymer blend may be immiscible, miscible, or compatible. Each of the two or more polyethylene polymers comprise greater than 50%, by weight, of its units derived from an ethylene monomer. This may include polyethylene homopolymers or copolymers (meaning units derived from two or more comonomers). In embodiments herein, the polyethylene polymer blend comprises at least 70 wt.% of the core layer. In some embodiments, the polyethylene polymer blend may comprise at least 75 wt.% of the core layer, at least 85 wt.% of the core layer, at least 95 wt.% of the core layer, at least 99 wt.% of the core layer, or 100 wt.% of the core layer.

The polyethylene polymer blend has an overall density of 0.910-0.945 g/cc. All individual values and subranges from 0.910-0.945 g/cc are included and disclosed herein. For example, in some embodiments, the polyethylene polymer blend has an overall density of 0.915 - 0.930 g/cc. In other embodiments, the polyethylene polymer blend has an overall density of 0.920 - 0.930 g/cc. In further embodiments, the polyethylene polymer blend has an overall density of 0.920 - 0.925 g/cc. Densities disclosed herein for ethylene-based polymers are determined according to ASTM D-792.

The polyethylene polymer blend has an overall melt index of 0.7-6 g/ 10 min. All individual values and subranges from 0.7-6 g/10 min are included and disclosed herein. For example, in some embodiments, the polyethylene polymer blend has a melt index of 2-6 g/10 min. In other embodiments, the polyethylene polymer blend has a melt index of 3-6 g/10 min. In further embodiments, the polyethylene polymer blend has a melt index of 4-6 g/10 min. Melt index, or $I_2$, for ethylene-based polymers is determined according to ASTM D1238 at 190° C, 2.16 kg.

The polyethylene polymer blend comprises at least 40%, by weight of the polyethylene polymer blend, of an ethylene-based polymer. In some embodiments, the polyethylene polymer blend comprises at least 50%, at least 55%, at least 60%, at least 65%, at least 70%, at least 75%, at least 80%, or at least 85%, by weight of the polyethylene polymer blend, of an ethylene-based polymer. The ethylene-based polymer has a polymer backbone that lacks measurable or demonstrable long chain branches. As used herein, "long chain branching" means branches having a chain length greater than that of any short chain branches, which are a result of comonomer incorporation. The long chain branch can be about the same length or as long as the length of the polymer backbone. In some embodiments, the ethylene-based polymer is substituted with an average of from 0.01 long chain branches/1000 carbons to 3 long chain branches/1000 carbons, from 0.01 long chain branches/1000 carbons to 1 long chain branches/1000 carbons, from 0.05 long chain branches/1000 carbons to 1 long chain branches/1000 carbons. In other embodiments, the ethylene-based polymer is substituted with an average of less than 1 long chain branches/1000 carbons, less than 0.5 long chain branches/1000 carbons, or less than 0.05 long chain branches/1000 carbons, or less than 0.01 long chain branches/1000 carbons. Long chain branching (LCB) can be determined by conventional techniques known in the industry, such as $^{13}C$ nuclear magnetic resonance ($^{13}C$ NMR) spectroscopy, and can be quantified using, for example, the method of Randall (Rev. Macromol. Chem. Phys., C29 (2 & 3), p. 285-297). Two other methods that may be used include gel permeation chromatography coupled with a low angle laser light scattering detector (GPC-LALLS), and gel permeation chromatography coupled with a differential viscometer detector (GPC-DV). The use of these techniques for long chain branch detection, and the underlying theories, have been well documented in the literature. *See, for example,* Zimm, B. H. and Stockmayer, W. H., J. Chem. Phys., 17, 1301 (1949) and Rudin A., Modern Methods of Polymer Characterization, John Wiley & Sons, New York (1991), pp. 103-112.

In some embodiments, the ethylene-based polymer may be a homogeneously branched or heterogeneously branched and/or unimodal or multimodal (e.g., bimodal) polyethylene. The ethylene-based polymer comprises ethylene homopolymers, copolymers of ethylene-derived units ("ethylene") and at least one type of comonomer, and blends thereof. Examples of suitable comonomers may include α-olefins. Suitable α-olefins may include those containing 3 to 20 carbon atoms (C3-C20). For example, the α-olefin may be a C4-C20 α-olefin, a C4-C12 α-olefin, a C3-C10 α-olefin, a C3-C8 α-olefin, a C4-C8 α-olefin, or a C6-C8 α-olefin. In some embodiments, the ethylene-based polymer is an ethylene/α-olefin copolymer, wherein the α-olefin is selected from the group consisting of propylene, 1-butene, 1-pentene, 1-hexene, 4-methyl-1-pentene, 1-heptene, 1-octene, 1-nonene and 1-decene. In other embodiments, the ethylene-based polymer is an ethylene/a-olefin copolymer, wherein the α-olefin is selected from the group consisting of propylene, 1-butene, 1-hexene, and 1-octene. In further embodiments, the ethylene-based polymer is an ethylene/a-olefin copolymer, wherein the α-olefin is selected from the group consisting of 1-hexene and 1-octene. In even further embodiments, the ethylene-based polymer is an ethylene/a-olefin copolymer, wherein the α-olefin is 1-octene. In even further embodiments, the ethylene-based polymer is a substantially linear ethylene/a-olefin copolymer, wherein the α-olefin is 1-octene. In some embodiments, the ethylene-based polymer is an ethylene/α-olefin copolymer, wherein the α-olefin is 1-butene.

The ethylene/a-olefin copolymers may comprise at least 50%, for example, at least 60%, at least 70%, at least 80%, at least 90%, at least 92%, at least 95%, at least 97%, by weight, of the units derived from ethylene; and less than 30%, for example, less than 25%, less than 20%, less than 15%, less than 10%, less than 5%, less than 3%, by weight, of units derived from one or more α-olefin comonomers.

Other examples of suitable ethylene-based polymers include substantially linear ethylene polymers, which are further defined in U.S. Pat. No. 5,272,236, U.S. Pat. No. 5,278,272, U.S. Pat. No. 5,582,923 and U.S. Pat. No. 5,733,155; homogeneously branched linear ethylene polymer compositions, such as those in U.S. Pat. No. 3,645,992; heterogeneously branched ethylene polymers, such as those prepared according to the process disclosed in U.S. Pat. No. 4,076,698; and/or blends thereof (such as those disclosed in U.S. Pat. No. 3,914,342 or U.S. Pat. No. 5,854,045). In some embodiments, the ethylene-based polymer may be a linear low density (LLDPE) polymer or substantially LLDPE polymer, and may include AFFINITY™ resins, ELITE™ resins, or ATTANE™ resins sold by The Dow Chemical Company, including ELITE™ 5230G resin, ATTANE™ 4404 resin, ATTANE™ 4202 resin, or AFFINITY™ 1840 resin; DOWLEX™ 2247 resin; or EXCEED™ resins sold by Exxon Mobil Corporation, including EXCEED™ 3518 resin or EXCEED™ 4518 resin; and EXACT™ resins sold by Exxon Mobil Corporation, including EXACT™ 3024.

The ethylene-based polymer can be made via gas-phase, solution-phase, or slurry polymerization processes, or any combination thereof, using any type of reactor or reactor configuration known in the art, e.g., fluidized bed gas phase reactors, loop reactors, stirred tank reactors, batch reactors in parallel, series, and/or any combinations thereof. In some embodiments, gas or slurry phase reactors are used. Suitable ethylene-based polymers may be produced according to the processes described at pages 15-17 and 20-22 in WO 2005/111291 A1. The catalysts used to make the ethylene-based polymer described herein may include Ziegler-Natta, metallocene, constrained geometry, or single site catalysts. In some embodiments, the ethylene-based polymer may be a LLDPE, such as, a znLLDPE, which refers to linear polyethylene made using Ziegler-Natta catalysts, a uLLDPE or "ultra linear low density polyethylene," which may include linear polyethylenes made using Ziegler-Natta catalysts, or a mLLDPE, which refers to LLDPE made using metallocene or constrained geometry catalyzed polyethylene.

The ethylene-based polymer has a density of 0.900 - 0.935 g/cc. All individual values and subranges from 0.900 - 0.935 g/cc are included and disclosed herein. For example, in some embodiments, the ethylene-based polymer has a density of 0.910 - 0.925 g/cc. In other embodiments, the ethylene-based polymer has a density of 0.900 - 0.920 g/cc. In further embodiments, the ethylene-based polymer has a density of 0.910 - 0.920 g/cc. Densities disclosed herein are determined according to ASTM D-792.

The ethylene-based polymer has a melt index, or $I_2$, of 0.7-6 g/10 min. All individual values and subranges from 0.7-6 g/10 min are included and disclosed herein. For example, in some embodiments, the ethylene-based polymer has a melt index of 2-5 g/10 min. In other embodiments, the ethylene-based polymer has a melt index of 2.5-4.5 g/10 min. Melt index, or $I_2$, for ethylene-based polymers is determined according to ASTM D1238 at 190° C, 2.16 kg.

The ethylene-based polymer disclosed herein may have a puncture resistance of greater than 8.3 $J/cm^3$ (100 ft·$lb_f$/$in^3$). All individual values and subranges of greater than 8.3 $J/cm^3$ (100 ft·$lb_f$/$in^3$) are included and disclosed herein.

For example, in some embodiments, the ethylene-based polymer has a puncture resistance of greater than 10.3 J/cm$^3$ (125 ft·lb$_f$/in$^3$). In other embodiments, the ethylene-based polymer has a puncture resistance of greater than 12.4 J/cm$^3$ (150 ft·lb$_f$/in$^3$). In further embodiments, the ethylene-based polymer has a puncture resistance of greater than 14.5 J/cm$^3$ (175 ft·lb$_f$/in$^3$). In even further embodiments, the ethylene-based polymer has a puncture resistance of greater than 16.5 J/cm$^3$ (200 ft·lb$_f$/in$^3$). Puncture resistance may be measured as described below in the test methods.

The ethylene-based polymer disclosed herein may have a spencer dart impact of greater than 100 g. All individual values and subranges of greater than 100 g are included and disclosed herein. For example, in some embodiments, the ethylene-based polymer has a spencer dart impact of greater than 115 g. In other embodiments, the ethylene-based polymer has a spencer dart impact of greater than 125 g. In further embodiments, the ethylene-based polymer has a spencer dart impact of greater than 135 g. In even further embodiments, the ethylene-based polymer has a spencer dart impact of greater than 150 g. Spencer dart impact may be measured as described below in the test methods.

In one embodiment, the ethylene-based polymer is a Ziegler-Natta catalyzed ethylene and octene copolymer, having a density from about 0.900 g/cc to about 0.925 g/cc. In another embodiment, the ethylene-based polymer is a single-site catalyzed LLDPE that is multimodal.

In embodiments herein, the polyethylene polymer blend may further comprise from 0 to 30%, by weight of the polyethylene polymer blend, of a low density polyethylene (LDPE). All individual values and subranges from 0 to 30% are included and disclosed herein. For example, in some embodiments, the polymer blend may further comprise from 5 to 20%, by weight of the polyethylene polymer blend, of a low density polyethylene. In other embodiments, the polymer blend may further comprise from 5 to 15%, by weight of the polyethylene polymer blend, of a low density polyethylene. In further, embodiments, the polymer blend may further comprise from 10 to 15%, by weight of the polyethylene polymer blend, of a low density polyethylene.

In embodiments herein, the LDPE present in the polyethylene polymer blend may have a density of about 0.915-0.930 g/cc. All individual values and subranges from 0.915-0.930 g/cc are included and disclosed herein. For example, in some embodiments, the LDPE has a density of 0.915 - 0.925 g/cc. In other embodiments, the LDPE has a density of 0.915 - 0.920 g/cc. In embodiments herein, the LDPE present in the polyethylene polymer blend has a melt index of 0.2-15 g/10 min. All individual values and subranges from 0.2-15 g/10 min are included and disclosed herein. For example, in some embodiments, the LDPE has a melt index of 1-12 g/10 min, preferably 2 to 12 g/10 min. In other embodiments, the LDPE has a melt index of 5-10 g/10 min.

The LDPE present in the polyethylene polymer blend may have a melt strength of greater than 5 cN. All individual values and subranges of greater than 5cN are included and disclosed herein. For example, in some embodiments, the LDPE has a melt strength of from 6-15 cN. In other embodiments, the LDPE has a melt strength of from 6-14 cN. In further embodiments, the LDPE has a melt strength of from 6-12 cN. In further embodiments, the LDPE has a melt strength of from 6-10 cN. In even further embodiments, the LDPE has a melt strength of from 6-18 cN.

The LDPE may include branched interpolymers that are partly or entirely homopolymerized or copolymerized in autoclave or tubular reactors at pressures above 100 MPa (14,500 psi) with the use of free-radical initiators, such as peroxides (see, for example U.S. Pat. No. 4,599,392). Examples of suitable LDPEs may include, but are not limited to, ethylene homopolymers, and high pressure copolymers, including ethylene interpolymerized with, for example, vinyl acetate, ethyl acrylate, butyl acrylate, acrylic acid, methacrylic acid, carbon monoxide, or combinations thereof. Exemplary LDPE resins may include resins sold by The Dow Chemical Company, such as, LDPE 722, LDPE 5004 and LDPE 621i. Other exemplary LDPE resins are described in WO 2005/023912.

In embodiments herein, the polyethylene polymer blend may further comprise from 0 to 30%, by weight of the polyethylene polymer blend, of a medium density polyethylene (MDPE) or a high density polyethylene (HDPE). All individual values and subranges from 0 to 30% are included and disclosed herein. For example, in some embodiments, the polymer blend may further comprise from 5 to 25%, by weight of the polyethylene polymer blend, of a medium or high density polyethylene. In other embodiments, the polymer blend may further comprise from 15 to 25%, by weight of the polyethylene polymer blend, of a medium or high density polyethylene. In further, embodiments, the polymer blend may further comprise from 20 to 25%, by weight of the polyethylene polymer blend, of a medium or high density polyethylene.

In embodiments herein, the MDPE or HDPE that may be present in the polyethylene polymer blend may have a density of about 0.930-0.965 g/cc. All individual values and subranges from 0.930-0.965 g/cc are included and disclosed herein. For example, in some embodiments, the MDPE or HDPE has a density of 0.940 - 0.965 g/cc. In other embodiments, the MDPE or HDPE has a density of 0.940 - 0.960 g/cc. In further embodiments, the MDPE or HDPE has a density of 0.945 - 0.955 g/cc. In embodiments herein, the MDPE or HDPE that may be present in the polyethylene polymer blend has a melt index of 0.7-10 g/10 min. All individual values and subranges from 0.7-10 g/10 min are included and disclosed herein. For example, in some embodiments, the MDPE or HDPE has a melt index of 1-10 g/10 min. In other embodiments, the MDPE or HDPE has a melt index of 3-8 g/10 min. In further embodiments, the MDPE or HDPE has a melt index of 5-7 g/10 min.

The MDPE or HDPE may be produced in various commercially available continuous reaction processes, particularly, those comprising two or more individual reactors in series or parallel using slurry, solution or gas phase process technology or hybrid reaction systems (e.g. combination of slurry and gas phase reactor). Exemplary processes may be found in U.S. Patent 4,076,698. Alternatively, the MDPE or HDPE polymers may also be produced by offline blending of 2 or more different polyethylene resins. For example, in some embodiments, a conventional mono-modal Ziegler-Natta MDPE or HDPE may be blended with a multi-modal Ziegler-Natta MDPE or HDPE. It is contemplated, however, that the various HDPE polymers can be produced with alternative catalyst systems, such as, metallocene, post-metallocene or chromium-based catalysts. Exemplary MDPE or HDPE resins may include resins sold by The Dow Chemical Company under the trade name HDPE 5962B, DMDA 8007 NT 7, AGILITY™ 6047G and DOWLEX™ 2027G.

In some embodiments, the polyethylene polymer blend comprises at least 70%, by weight of a polyethylene polymer blend, of an ethylene-based polymer having a density of 0.900-0.925 g/cc and a melt index of 0.7-6 g/10 min, and further comprises 5% to 15%, by weight of the polyethylene polymer blend, of a LDPE having a density of about 0.915-0.930 g/cc and a melt index of about 1-15 g/10 min. In other embodiments, the polyethylene polymer blend comprises at least 70%, by weight of the polyethylene polymer blend, of an ethylene-based polymer having a density of 0.900-0.925 g/cc and a melt index of 0.7-6 g/10 min, preferably 1-6 g/10 min and further comprises 15% to 25%, by weight of the polyethylene polymer blend, of a medium or high density polyethylene having a density of about 0.930-0.965 g/cc and a melt index of about 1-10 g/10 min. In further embodiments, the polyethylene polymer blend comprises at least 70%, by weight of the polyethylene polymer blend, of an ethylene-based polymer having a density of 0.900-0.925 g/cc and a melt index of 0.7-6 g/10 min, and further comprises 5% to 15%, by weight of the polyethylene polymer blend, of a LDPE having a density of about 0.915-0.930 g/cc and a melt index of about 1-15 g/10 min, and 15% to 25%, by weight of the polyethylene polymer blend, of a medium or high density polyethylene having a density of about 0.930-0.965 g/cc and a melt index of about 1-10 g/10 min. Of course, it should be understood that the foregoing amounts, density ranges, and melt index ranges are exemplary, and other amounts, density ranges, and melt index ranges as previously described herein, may be incorporated into various embodiments herein.

In embodiments herein, the polyethylene polymer blend may be formed by a variety of methods. For example, it may be made by blending or mixing the polymer components together. Blending or mixing can be accomplished by any suitable mixing means known in the art, including melt or dry/physical blending of the individual components. Alternatively, the polyethylene polymer blend may be made in a single reactor or a multiple reactor configuration, where the multiple reactors may be arranged in series or parallel, and where each polymerization takes place in solution, in slurry, or in the gas phase. It should be understood that other suitable methods for blending or mixing the polymer components together may be utilized.

The core layer may optionally comprise one or more additives. Such additives may include, but are not limited to, antioxidants (e.g., hindered phenolics, such as, IRGANOX@ 1010 or IRGANOX@ 1076, supplied by Ciba Geigy), phosphites (e.g., IRGAFOS® 168, also supplied by Ciba Geigy), cling additives (e.g., PIB (polyisobutylene)), Standostab PEPQ™ (supplied by Sandoz), pigments, colorants, fillers (e.g., calcium carbonate, talc, mica, kaolin, perlite, diatomaceous earth, dolomite, magnesium carbonate, calcium sulfate, barium sulfate, glass beads, polymeric beads, ceramic beads, natural and synthetic silica, aluminum trihydroxide, magnesium trihydroxide, wollastonite, whiskers, wood flour, lignine, starch), $TiO_2$, anti-stat additives, flame retardants, biocides, antimicrobial agents, and clarifiers/nucleators (e.g., HYPERFORM™ HPN-20E, MILLAD™ 3988, MILLAD™ NX 8000, available from Milliken Chemical). The one or more additives can be included in the polyethylene polymer blend at levels typically used in the art to achieve their desired purpose. In some examples, the one or more additives are included in amounts ranging from 0-10 wt.% of the polyethylene polymer blend, 0-5 wt.% of the polyethylene polymer blend, 0.001-5 wt.% of the polyethylene polymer blend, 0.001-3 wt.% of the polyethylene polymer blend, 0.05-3 wt.% of the polyethylene polymer blend, or 0.05-2 wt.% of the polyethylene polymer blend.

Skin Layers

The skin layers do not contain non-woven materials. Each skin layer independently comprises a propylene-based polymer. The propylene-based polymer may be a propylene homopolymer, or a polypropylene polymer blend. The propylene-based polymer comprises a majority weight percent of polymerized propylene monomer (based on the total amount of polymerizable monomers), and optionally, one or more comonomers.

The propylene homopolymer may be isotactic, atactic or syndiotactic. In some embodiments, the propylene homopolymer is isotactic. Each skin layer may independently comprise 100 wt.% of the propylene homopolymer, excluding additives, as discussed further below.

As used herein, "polypropylene polymer blend" refers to a mixture containing greater than 50 wt.% of a propylene-based polymer. The components of the polypropylene polymer blend may be immiscible, miscible, or compatible with each other. In some embodiments, each skin layer may independently comprise at least 55 wt.% of the polypropylene polymer blend, at least 60 wt.% of the polypropylene polymer blend, at least 65 wt.% of the polypropylene polymer blend, at least 75 wt.% of the polypropylene polymer blend, at least 80 wt.%, at least 90 wt.%, at least 95 wt.%, at least 99 wt.%, or 100 wt.% of the polypropylene polymer blend.

As stated above, the polypropylene polymer blend comprises greater than 50 wt.%, by weight of the polypropylene polymer blend, of a propylene-based polymer. In some embodiments, the polypropylene polymer blend comprises greater than 55 wt.%, greater than 60 wt.%, greater than 65 wt.%, by weight of the polypropylene polymer blend, of a propylene-based polymer.

The propylene copolymer may be a propylene/olefin copolymer (random or block) or a propylene impact copolymer. Impact propylene copolymers may also include heterophasic propylene copolymers, where polypropylene is the continuous phase and an elastomeric phase is uniformly dispersed therein. For polypropylene/olefin copolymers, nonlimiting examples of suitable olefin comonomers include ethylene, C4-C20 $\alpha$-olefins, such as 1-butene, 1-pentene, 1-hexene, 4-methyl-1-pentene, 1-heptene, 1-octene, 1-decene, or 1-dodecene; C4-C20 diolefins, such as 1,3-butadiene, 1,3-pentadiene, norbornadiene, 5-ethylidene-2-norbornene (ENB) and dicyclopentadiene; C8-C40 vinyl aromatic compounds, such as styrene, o-, m-, and p-methylstyrene, divinylbenzene, vinylbiphenyl, vinylnapthalene; and halogen-substituted $C_8$-$C_{40}$ vinyl aromatic compounds, such as chlorostyrene and fluorostyrene. In some embodiments, the propylene copolymers include propylene/ethylene, propylene/1-butene, propylene/1-hexene, propylene/4-methyl-1-pentene, propylene/1-octene, or propylene/ethylene/1-butene.

Suitable polypropylenes are formed by means within the skill in the art, for example, using Ziegler-Natta catalysts, a single-site catalysts (metallocene or constrained geometry), or non-metallocene, metal-centered, heteroaryl ligand catalysts. Exemplary propylene-based polymer resins may include PP 3155 commercially available from the Exxon Mobil Corporation, USA, polypropylene 6231, commercially available from LyondellBasell Industries, USA or resins sold under the trade name VERSIFY™ commercially available from The Dow Chemical Company, USA, VISTAMAXX™ (commercially available from ExxonMobil Chemical Company) propylene polymers commercially available from Braskem under various tradenames and/or trademarks, PROFAX® (commercially available from Lyondell Basell) ), or Borealis BORSOFT™ (commercially available from Borealis of Denmark).

In embodiments herein, the propylene-based polymer has a melt flow rate (MFR) from 0.1 g/10 min to 100 g/10 min. All individual values and subranges from 0.1 g/10 min to 100 g/10 min are included and disclosed herein. For example, in some embodiments, the propylene-based polymer has a melt flow rate from 1 g/10 min to 75 g/10 min, from 2 g/10 min to 50 g/10 min, from 10 g/10 min to 45 g/10 min, or from 15 g/10 min to 40 g/10 min, as measured in accordance with ASTM D1238 (230°C, 2.16 kg). In embodiments herein, the propylene-based polymer has a density of 0.890 to 0.920 g/cc. All individual values and subranges from 0.890 to 0.920 g/cc are included and disclosed herein. For example, in some embodiments, propylene-based polymer has a density of 0.900 to 0.920 g/cc, or from 0.89 to 0.915 g/cc. The density may be determined according to ASTM D-792.

The propylene-based polymer may have a 2% secant modulus of greater than 103421 kPa (15,000 psi). The 2% secant modulus is an average of the secant modulus in the machine direction (MD) and the cross direction (CD), and may be calculated as follows:

$$2\% \; secant \; modulus = \frac{(2\% \; secant \; modulus(MD) + 2\% \; secant \; modulus \; (CD))}{2}$$

All individual values and subranges greater than 103421 kPa (15,000 psi) are included and disclosed herein. For example, in some embodiments, the propylene-based polymer has a 2% secant modulus of greater than 120658 kPa (17,500 psi). In other embodiments, the propylene-based polymer has a 2% secant modulus of greater than 137895 kPa (20,000 psi). In further embodiments, the propylene-based polymer has a 2% secant modulus of greater than 189605 kPa (27,500 psi). In even further embodiments, the propylene-based polymer has a 2% secant modulus of greater than 241317 kPa (35,000 psi). In even further embodiments, the propylene-based polymer has a 2% secant modulus of from 103421 kPa (15,000 psi) to 344738 kPa (50,000 psi). In even further embodiments, the propylene-based polymer has a 2% secant modulus of from 172369 kPa (25,000 psi) to 310264 kPa (45,000 psi). In even further embodiments, the propylene-based polymer has a 2% secant modulus of from 206843 kPa (30,000 psi) to 310264 kPa (45,000 psi). The 2% secant modulus may be determined according to ASTM 882.

The polypropylene polymer blend comprises a low density polyethylene (LDPE). The polypropylene polymer blend independently comprises 10 wt.% to 30 wt.%, or 15 wt.% to 25wt.% of the LDPE. In an embodiment, the LDPE present in the polypropylene polymer blend has a density of about 0.915-0.930 g/cc. All individual values and subranges from 0.915-0.930 g/cc are included and disclosed herein. For example, in some embodiments, the LDPE has a density of 0.915 - 0.925 g/cc. In other embodiments, the LDPE has a density of 0.915 - 0.920 g/cc. In embodiments herein, the LDPE present in the skin layers has a melt index of 1-15 g/10 min. All individual values and subranges from 1-15 g/10 min are included and disclosed herein. For example, in some embodiments, the LDPE has a melt index of 2-12 g/10 min. In other embodiments, the LDPE has a melt index of 5-10 g/10 min.

The LDPE present in the polypropylene polymer blend may have a melt strength of greater than 5 cN. All individual values and subranges of greater than 5cN are included and disclosed herein. For example, in some embodiments, the LDPE has a melt strength of from 6-15 cN. In other embodiments, the LDPE has a melt strength of from 6-14 cN. In further embodiments, the LDPE has a melt strength of from 6-12 cN. In further embodiments, the LDPE has a melt strength of from 6-10 cN. In even further embodiments, the LDPE has a melt strength of from 6-18 cN.

LDPEs present in the polypropylene polymer blend may include branched polymers that are partly or entirely homopolymerized or copolymerized in autoclave or tubular reactors at pressures above 100 MPa (14,500 psi) with the use of free-radical initiators, such as peroxides *(see* for example U.S. Pat. No. 4,599,392). Examples of suitable LDPEs present in the polypropylene polymer blend may include, but are not limited to, ethylene homopolymers, and high pressure copolymers, including ethylene interpolymerized with, for example, vinyl acetate, ethyl acrylate, butyl acrylate, acrylic acid, methacrylic acid, carbon monoxide, or combinations thereof. Exemplary LDPE resins may include resins sold by The Dow Chemical Company, such as, LDPE 722, LDPE 5004, and LDPE 621i. Other exemplary LDPE resins are described in WO 2005/023912.

The polypropylene polymer blend may further comprise a compatibilizer agent capable of compatibilizing blends of polyethylene and polypropylene polymers. Suitable compatibilizer agents include olefin plastomers and elastomers, such as, ethylene-based and propylene-based copolymers available under the trade name VERSIFY™(from The Dow Chemical Company), SURPASS™ (from Nova Chemicals), and VISTAMAXX™(from Exxon Mobil Corporation). Exemplary compatibilizers may include the VERSIFY™3401 compatibilizer (from The Dow Chemical Company), the VISTAMAXX™6202 compatibilizer (from Exxon Mobil Corporation) ), or Borealis BORSOFT™ (commercially available from Borealis of Denmark),

Each skin layer may independently comprise one or more additives. Such additives may include, but are not limited to, antioxidants (e.g., hindered phenolics, such as, IRGANOX@ 1010 or IRGANOX@ 1076, supplied by Ciba Geigy), phosphites (e.g., IRGAFOS® 168, also supplied by Ciba Geigy), cling additives (e.g., PIB (polyisobutylene)), Standostab PEPQ™ (supplied by Sandoz), pigments, colorants, fillers (e.g., calcium carbonate, mica, talc, kaolin, perlite, diatomaceous earth, dolomite, magnesium carbonate, calcium sulfate, barium sulfate, glass beads, polymeric beads, ceramic beads, natural and synthetic silica, aluminum trihydroxide, magnesium trihydroxide, wollastonite, whiskers, wood flour, lignine, starch), $TiO_2$, anti-stat additives, flame retardants, slip agents, antiblock additives, biocides, antimicrobial agents, and clarifiers/nucleators (e.g., HYPERFORM™ HPN-20E, MILLAD™ 3988, MILLAD™ NX 8000, available from Milliken Chemical). The one or more additives can be included in the polypropylene polymer blend at levels typically used in the art to achieve their desired purpose. In some examples, the one or more additives are included in amounts ranging from 0-10 wt.% of the polypropylene polymer blend, 0-5 wt.% of the polypropylene polymer blend, 0.001-5 wt.% of the polypropylene polymer blend, 0.001-3 wt.% of the polypropylene polymer blend, 0.05-3 wt.% of the polypropylene polymer blend, or 0.05-2 wt.% of the polypropylene polymer blend.

Multilayer Films

The multilayer films described herein may be coextruded films. In some embodiments, the multilayer film is a coextruded film, whereby at least one of the skin layers is coextruded to the core layer. In other embodiments, the multilayer film is a coextruded film, whereby one of the skin layers (i.e., a first skin layer) is coextruded to the core layer and the other skin layer (i.e., a second skin layer) is coextruded to the core layer, and the two coextruded films are laminated together such that the core layer is positioned between the two skin layers. In further embodiments, the multilayer film is a coextruded film, whereby the skin layers are coextruded to the core layer.

Films may be made via any number of processes including cast film where the polymer is extruder through a flat die to create a flat film or blown film whereby the polymer is extruded through an annular die and creates a tube of film that can be slit to create the flat film.

In embodiments herein, the multilayer film may have a basis weight of between about 10-20 gsm. All individual values and subranges from 10-20 gsm are included and disclosed herein. For example, in some embodiments, the multilayer film may have a basis weight of between about 10-18 gsm. In other embodiments, the multilayer film may have a basis weight of between about 10-16 gsm. In further embodiments, the multilayer film may have a basis weight of between about 10-14 gsm.

In some embodiments, the multilayer films described herein may exhibit at least one of the following properties: a spencer dart impact of greater than about 160 g (or, alternatively, greater than 170 g or 180 g); a secant modulus at 2% of greater than about 110316 kPa (16,000 psi) in the MD (or alternatively, greater than 117211 kPa (17,000 psi) or 124106 kPa (18,000 psi)) and greater than 110316 kPa (16, 000 psi) in the CD (or alternatively, greater than 117211 kPa (17,000 psi)); a stress at break (also called load at break) in the cross-direction of greater than about 11721 kPa (1,700 psi) (or, alternatively, greater than about 12411 kPa (1,800 psi) or 13100 kPa (1,900 psi)), and in the machine direction of greater than about 13790 kPa (2,000 psi) (or, alternatively, greater than about 14479 kPa (2,100 psi), 15168 kPa (2,200 psi), or 15858 kPa (2,300 psi)); or a puncture resistance greater than about 2.45 $J/cm^3$ (30 $ft \cdot lb_f/in^3$) (or, alternatively, 2.90 $J/cm^3$ (35 $ft \cdot lb_f/in^3$) or 3.31 $J/cm^3$ (40 $ft \cdot lb_f/in^3$)). In some embodiments, the multilayer films described herein may exhibit at least one of the following properties: a softness value difference of less than 5%, when compared to a 100% polyethylene film having a 2% secant modulus greater than about 110316 kPa (16,000 psi) in the MD, or a noise value of less than 0.5 dB between a frequency band of 1,000 Hz and 5,000 Hz. The Softness Value Difference (SVD) may be calculated as follows:

$$SVD = \frac{|Softness\ Value\ (inventive\ film) - Softness\ Value\ (reference\ film)|}{Softness\ Value\ (reference\ film)}\ x\ 100\%$$

wherein the reference film is a 100% polyethylene film having a 2% secant modulus of greater than 110316 kPa (16,000 psi). As used herein a "100% polyethylene film" refers to a film consisting of one or more polymers that contain more than 50 mole percent polymerized ethylene monomer (based on the total amount of polymerizable monomers) and, optionally, may contain at least one comonomer. Without being bound by theory, it is believed that one or more of the properties result from improved film structure and improved component amounts in each layer of the film structure such that key attributes of each material are incorporated. In particular, it is believed that incorporating particular amounts of polypropylene into the skin layers can assist in adhesion, while selecting a particular polyethylene blend in the core layer can avoid pinholes that may form between polypropylene substrates and polyethylene films, while still providing adequate strength and modulus necessary for a backsheet. It is also believed that by selecting certain polyethylene polymers for incorporation into the core and skin layers, the haptics properties, in particular, noise and softness, can be improved.

Laminates

Also described herein are ultrasonically-bonded laminates. The ultrasonically-bonded laminates comprise a multilayer film as previously described herein, and a nonwoven substrate at least partially ultrasonically bonded to the multilayer film. As used herein, "nonwoven substrates" include nonwoven webs, nonwoven fabrics and any nonwoven structure in which individual fibers or threads are interlaid, but not in a regular or repeating manner. Nonwoven substrates described herein may be formed by a variety of processes, such as, for example, air laying processes, meltblowing processes, spunbonding processes and carding processes, including bonded carded web processes. As used herein, "ultrasonicbonding" includes ultrasonic welding.

The nonwoven web may comprise a single web, such as a spunbond web, a carded web, an airlaid web, a spunlaced web, or a meltblown web. However, because of the relative strengths and weaknesses associated with the different processes and materials used to make nonwoven fabrics, composite structures of more than one layer are often used in order to achieve a better balance of properties. Such structures are often identified by letters designating the various lays such as SM for a two layer structure consisting of a spunbond layer and a meltblown layer, SMS for a three layer structure, or more generically $SX_nS$ structures, where X can be independently a spunbond layer, a carded layer, an airlaid layer, a spunlaced layer, or a meltblown layer and n can be any number, although for practical purposes is generally less than 5. In order to maintain structural integrity of such composite structures, the layers must be bonded together. Common methods of bonding include point bonding, adhesive lamination, and other methods known to those skilled in the art. All of these structures may be used in the present invention.

The fibers which make up the nonwoven web are monocomponent fibers. It is preferred that the surface of the fiber comprise a polyethylene resin other than LDPE. The polyethylene resin can advantageously be a single site catalyzed resin (mLLDPE), or a post metallocene catalyzed LLDPE, or a Ziegler-Natta catalyzed LLDPE, or HDPE, or MDPE. If monocomponent fibers are used it is preferred that the resin used in the fiber comprise 100% linear (including "substantially linear") polyethylene.

In embodiments herein, the nonwoven substrate is made from a propylene-based material, 100% polyethylene, or polyethylene/polypropylene blends. Bi-component structures such as skin-core structures will not be used as a substrate. Examples of suitable propylene-based materials include materials that comprise a majority weight percent of polymerized propylene monomer (based on the total amount of polymerizable monomers), and optionally, one or more comonomers. This may include propylene homopolymer (i.e., a polypropylene), a propylene copolymer, or combinations thereof. The propylene copolymer may be a propylene/olefin copolymer. Nonlimiting examples of suitable olefin comonomers include ethylene, C4-C20 $\alpha$-olefins, such as 1-butene, 1-pentene, 1-hexene, 4-methyl-1-pentene, 1-heptene, 1-octene, 1-decene, or 1-dodecene. In some embodiments, the propylene-based material is polypropylene homopolymer.

The nonwoven substrate may comprise one or more layers. The one or more layers may be spunbond non-woven layers (S), meltblown non-woven layers (M), wet-laid non-woven layers, air-laid non-woven layers, webs produced by any non-woven or melt spinning process. In some embodiments, the nonwoven substrate comprises at least one spunbond layer (S) and at least one meltblown layer (M). In other embodiments, the nonwoven substrate comprises at least one spunbond layer (S) and at least one meltblown layer (M), and may have one of the following structures: SSS, SM, SMS, SMMS, SSMMS, or SSMMMS. The outermost spunbond layer may comprise a material selected from the group consisting of spunbond homopolymer polypropylene (hPP), spunbond heterogeneously branched polyethylene, or carded hPP.

The ultrasonically-bonded laminates described herein may exhibit at least one of the following properties: a peel force value of greater than about 1.2 N, or a hydrostatic pressure above 7 kPa (70 mbar). Without being bound by theory, it is believed that incorporating particular amounts of polypropylene into the skin layers can assist in adhesion and selecting a particular polyethylene blend for the core layer can avoid or reduce pinholes during an ultrasonic bonding process between polypropylene substrates and polyethylene-based films. Having a higher level of pinholes can allow more water to pass through the laminate resulting in a lower hydrostatic pressure, while having a lower level of pinholes can result in a higher hydrostatic pressure due to less water passing through.

End Uses

The films or ultra-sonically bonded laminates described herein may be used in a variety of applications. In some embodiments, the films or laminates can be used in hygiene applications, such as diapers, training pants, and adult incontinence articles, or in other similar absorbent garment applications. In other embodiments, the films or laminates can be used in medical applications, such as medical drapes, gowns, and surgical suits, or in other similar fabric (woven or nonwoven) applications.

The films or laminates may be breathable or non-breathable. As used herein, the term "breathable" refers to a material which is permeable to water vapor. The water vapor transmission rate (WVTR) or moisture vapor transfer rate (MVTR) is measured in grams per square meter per 24 hours, and shall be considered equivalent indicators of breathability. The term "breathable" refers to a material which is permeable to water vapor having a minimum WVTR (water vapor transmission rate) of greater than about 100 $g/m^2/24$ hours. In some embodiments, the breathability is greater than about 300 $g/m^2/24$ hours. In other embodiments, the breathability is greater than about 500 $g/m^2/24$ hours. In further embodiments, the breathability is greater than about 1000 $g/m^2/24$ hours.

The WVTR of films or laminates, in one aspect, gives an indication of how comfortable the article would be to wear. Often, hygiene applications of breathable films or laminates desirably have higher WVTRs and films or laminates of the present invention can have WVTRs exceeding about 1,200 $g/m^2/24$ hours, 1,500 $g/m^2/24$ hours, 1,800 $g/m^2/24$ hours or even exceeding 2,000 $g/m^2/24$ hours. A suitable technique for determining the WVTR (water vapor transmission rate) value of a film or laminate material of the invention is the test procedure standardized by INDA (Association of the Nonwoven Fabrics Industry), number IST-70.4-99, entitled "STANDARD TEST METHOD FOR WATER VAPOR TRANSMISSION RATE THROUGH NONWOVEN AND PLASTIC FILM USING A GUARD FILM AND VAPOR PRESSURE SENSOR". The INDA procedure provides for the determination of WVTR, the permeance of the film to water vapor and, for homogeneous materials, water vapor permeability coefficient.

Breathable films may be obtained by adding fillers, like $CaCO_3$, clay, silica, alumina, talc, etc., to make moisture breathable films of high WVTR, which requires a postorientation process, such as machine direction orientation or the use of inter-digitating or inter-meshing rollers, also called "ring rolling", to create cavitation around the filler particles (see, for example, WO2007/081548 or WO1998/004397). Enhanced moisture permeation in such films is a result of microporous morphology. Such films are commonly used hygiene applications for diaper and adult incontinence backsheet films and in medical applications such as breathable but liquid impermeable surgical gowns and can yield WVTR values of greater than 500 $g/m^2/24$ hours up to 20,000 $g/m^2/24$ hours, depending upon the level of $CaCO_3$ and stretching, for films ranging in thickness from 0.0051 to 0.038 mm (0.2 to 1.5 mils) thickness.

**TEST METHODS**

Unless otherwise stated, the following test methods are used. All test methods are current as of the filing date of this disclosure.

Density

Densities disclosed herein for ethylene-based and propylene-based polymers are determined according to ASTM D-792.

Melt Index

Melt index, or $I_2$, for ethylene-based polymers is determined according to ASTM D1238 at 190°C, 2.16 kg.

Melt Flow Rate

Melt Flow Rate, or MFR, for propylene-based polymers is measured in accordance with ASTM D1238 at 230°C, 2.16 kg.

Melt Strength

Melt Strength measurements are conducted on a Gottfert Rheotens 71.97 (Göettfert Inc.; Rock Hill, SC) attached to a Gottfert Rheotester 2000 capillary rheometer. A polymer melt (about 20-30 grams, pellets) is extruded through a capillary die with a flat entrance angle (180 degrees) with a capillary diameter of 2.0 mm and an aspect ratio (capillary length/capillary diameter) of 15. After equilibrating the samples at 190°C for 10 minutes, the piston is run at a constant piston speed of 0.265 mm/second. The standard test temperature is 190°C. The sample is drawn uniaxially to a set of accelerating nips located 100 mm below the die, with an acceleration of 2.4 mm/second2. The tensile force is recorded as a function of the take-up speed of the nip rolls. Melt strength is reported as the plateau force (cN) before a strand breaks. The following conditions are used in the melt strength measurements: plunger speed = 0.265 mm/second; wheel acceleration = 2.4 mm/s2; capillary diameter = 2.0 mm; capillary length = 30 mm; and barrel diameter = 12 mm.

2% Secant Modulus/Stress at Break

Tensile properties, including the secant modulus at 2% strain and the stress at break, are determined in the machine and cross directions according to ASTM D882.

Spencer Dart Impact Strength

The Spencer dart test is determined according to ASTM D3420, Procedure B.

Peel Force

Films are ultrasonically bonded to a nonwoven to form a laminate. The specimen size is 127 mm X 25.4 mm. Five specimens are measured per laminate. Peel force is determined by separating the film from a nonwoven substrate, and is a measure of the energy required to separate the layers per unit area. At a first end of the specimen, 2.54 cm (one inch) of the film is manually separated from the nonwoven substrate to form a starting gap. The film is placed in the movable grip of a CRE tensile testing machine (Instron) while the nonwoven substrate is placed in a stationary 180° plane. The films are peeled from the nonwoven substrate at a rate of about 304.8 mm/min.

Puncture Resistance

Puncture is measured on a tensile testing machine according to ASTM D5748, except for the following: square specimens are cut from a sheet to a size of 15.24 cm (6 inches) by 15.24 cm (6 inches); the specimen is clamped in a 10.16 cm (4 inch) diameter circular specimen holder and a puncture probe is pushed into the center of the clamped film at a cross head speed of 25.4 cm/minute (10 inches/minute); the probe is a 1.27 cm (0.5 inch) diameter polished steel ball on a 0.635 cm (0.25 inch) support rod; there is a 19.56 (7.7 inch) maximum travel length to prevent damage to the test fixture; there is no gauge length - prior to testing, the probe is as close as possible to, but not touching, the specimen. A single thickness measurement is made in the center of the specimen. A total of five specimens are tested to determine an average puncture value.

Noise

Noise tester equipment includes an acoustic isolated box that contains a microphone MK 221 used to capture sound and a NC 10 Audio Acoustic Analyzer by Neutrix Cortex Instruments. The microphone is sensitive to a signal having a Frequency (Hz) of 20 Hz - 20,000 Hz. The microphone is located in the center of the acoustic box at 10 cm horizontally aligned with the film surface and 25 cm vertically aligned with the box top. The acoustic isolated box is made of lead with dimensions of 53 cm x 53 cm x 53 cm. Films are cut to a specimen size of 10 cm X 10 cm. The specimen is fixed to two holders, a first holder that is stationary and a second holder that is movable to provide a flexing motion of the film. The equipment is run in vacuum to obtain ground-noise readings that are subtracted from noise readings generated by each specimen. The data is collected on the 1/3 octave. Four different specimens are measured per film.

Softness

The "softness" or "hand" quality is considered to be the combination of resistance due to surface friction, flexibility, and compressibility of a fabric material. A Handle-O-Meter tester (manufactured by Thwing-Albert Instrument Co., West Berlin, N.J.) measures the above factors using a Linear Variable Differential Transformer (LVDT) to detect the resistance that a blade encounters when forcing a specimen of material into a slot of parallel edges. Samples are cut into 8 in x 8 in square specimens. The Handle-O-Meter slot width is set at 20 mm. Measurements are taken in each of four positions per specimen as required by the instrument manufacturer's test manual, and the four measurements are summed to give the total hand for a single specimen in grams-force. This averaged hand is then normalized to the specimen weight and volume. Samples having a lower resistance value are considered to have better softness.

Hydrostatic Pressure

The hydrostatic pressure is measured according to ISO 1420. The equipment used is a hydrostatic head tester (FX 3000, TexTest AG, Switzerland). The test specimens are 15 cm x 15 cm squares, the test area is 100 cm$^2$, and the distilled water temperature was set to 20 +/- 2°C. The results are expressed in mbar/min.

**EXAMPLES**

The embodiments described herein may be further illustrated by the following non-limiting examples.

Three layer films were made as outlined below. The films were produced on a three layer commercial cast line having a maximum line speed of 200 m/min, a melt temperature of 260°C, a die temp of 260°C, a die gap of 0.0203 mm (0.8 mils), and an air gap of 22.9 cm (9 in). The multilayer films have a basis weight of 14 gsm. The core layer comprises 70% of the overall film thickness. Each skin layer comprises 15% of the overall film thickness.

**Preparation of Inventive Film**

The Inventive Example used the following resins: a low density polyethylene (LDPE) is a high pressure low density polyethylene made in an autoclave reactor having has a density of 0.918 g/cc and a melt index of 8.0 g/10 min (LDPE 722 from The Dow Chemical Company, USA); an isotactic polypropylene homopolymer having a density of 0.900 g/cc and a melt flow rate of 22 g/10 min (Polypropylene 6231, available from LyondellBasell Industries, USA); an ethylene-based polymer that is an ethylene-octene copolymer having a density of 0.916 g/cc and a melt index of 4.0 g/10 min (ELITE™ 5230G from The Dow Chemical Company, USA); and a medium or high density polyethylene (MDPE or HDPE) that is an ethylene-octene copolymer having a density of 0.947 g/cc and a melt index of 6.0 g/10 min (AGILITY™ 6047G from The Dow Chemical Company, USA). The multilayer films were ultrasonically bonded using a VE 20 MICROBOND CSI ultrasound device.

| Inventive Example | Skin (wt.%) | Core (wt.%) | Skin (wt.%) |
|---|---|---|---|
| LDPE | 20 | 10 | 20 |
| Polypropylene | 80 | 0 | 80 |
| Ethylene-B ased Polymer | 0 | 70 | 0 |
| MDPE/HDPE | 0 | 20 | 0 |

**Preparation of Comparative Films**

Comparative Example 1 is an isotactic polypropylene homopolymer having a density of 0.900 g/cc and a melt flow rate of 22 g/10 min (Polypropylene 6231, available from LyondellBasell Industries, USA).

| Comparative Example 1 | Skin (wt.%) | Core (wt.%) | Skin (wt.%) |
|---|---|---|---|
| Polypropylene | 100 | 100 | 100 |

Comparative Example 2 is an isotactic polypropylene homopolymer having a density of 0.900 g/cc and a melt flow rate of 22 g/10 min (Polypropylene 6231, available from LyondellBasell Industries, USA) and a high pressure low density polyethylene made in an autoclave reactor having a density of 0.918 g/cc and a melt index of 8.0 g/10 min (LDPE 722 from The Dow Chemical Company, USA).

| Comparative Example 2 | Skin (wt.%) | Core (wt.%) | Skin (wt.%) |
|---|---|---|---|
| LDPE | 15 | 15 | 15 |
| Polypropylene | 85 | 85 | 85 |

Comparative Example 3 is a high pressure low density polyethylene made in an autoclave reactor having a density of 0.918 g/cc and a melt index of 8.0 g/10 min (LDPE 722 from The Dow Chemical Company, USA), and a medium or high density polyethylene (MDPE/HDPE) having a density of 0.947 g/cc and a melt index of 6.0 g/10 min (AGILITY™ 6047G from The Dow Chemical Company, USA).

| Comparative Example 3 | Skin (wt.%) | Core (wt.%) | Skin (wt.%) |
|---|---|---|---|
| LDPE | 15 | 15 | 15 |
| MDPE/HDPE | 85 | 85 | 85 |

Comparative Example 4 is a high pressure low density polyethylene made in an autoclave reactor having a density of 0.918 g/cc and a melt index of 8.0 g/10 min (LDPE 722 from The Dow Chemical Company, USA); an ethylene-based polymer that is an ethylene-octene copolymer having a density of 0.916 g/cc and a melt index of 4.0 g/10 min (ELITE™ 5230G from The Dow Chemical Company, USA); and a medium or high density polyethylene (MDPE/HDPE) having a density of 0.947 g/cc and a melt index of 6.0 g/10 min (AGILITY™ 6047G from The Dow Chemical Company, USA).

| Comparative Example 4 | Skin (wt.%) | Core (wt.%) | Skin (wt.%) |
|---|---|---|---|
| LDPE | 15 | 15 | 15 |
| MDPE/HDPE | 65 | 65 | 65 |
| Ethylene-based polymer | 25 | 25 | 25 |

**Preparation of Laminates**

The inventive and comparative films are point bonded using ultrasonic bonding to a spunbond polypropylene nonwoven having a basis weight of 14 gsm. About 9% of the area is bonded. The line speed was 200 m/min, the welding force was 700-1150 N, and the frequency was 90%.

Results

Table 1

| | Comparative film 1 | Comparative film 2 | Comparative film 3 | Comparative film 4 | Inventive example |
|---|---|---|---|---|---|
| | | | | | |
| Noise Intensity, dB (Freq. Range 20-20,000 Hz) | 32.47 | 28.21 | 22.07 | 5.72 | 0.41 |
| Softness, g | 4.55 | 3.25 | 2.10 | 2.20 | 2.00 |
| Puncture resistance, $J/cm^3$ ($ft*lb_f/in^3$) | 0.44 (5.36) | 0.41 (4.93) | 0.51 (6.21) | 1.09 (13.17) | 3.36 (40.56) |
| Spencer Dart Impact, g | 68.39 | 72.40 | 94.00 | 117.20 | 186.30 |

(continued)

| Softness, g | 4.55 | 3.25 | 2.10 | 2.20 | 2.00 |
|---|---|---|---|---|---|
| 2% Secant Modulus CD, kPa (psi) | 253790 (36809.88) | 206441 (29941.67) | 170672 (24753.83) | 114292 (16576.68) | 131824 (19119.41) |
| 2% Secant Modulus MD, kPa (psi) | 264931 (38424.96) | 192091 (27860.48) | 167879 (24348.85) | 132107 (19160.56) | 133633 (19381.85) |
| Load @ Break CD, kPa (psi) | 11548 (1674.91) | 4899 (710.59) | 11042 (1601.55) | 12481 (1810.21) | 13695 (1986.25) |
| Load @ Break MD, kPa (psi) | 11553 (1675.55) | 9852 (1428.90) | 11602 (1682.73) | 14907 (2162.15) | 15928 (2310.18) |
| 2% Secant Modulus Results | | | | | |

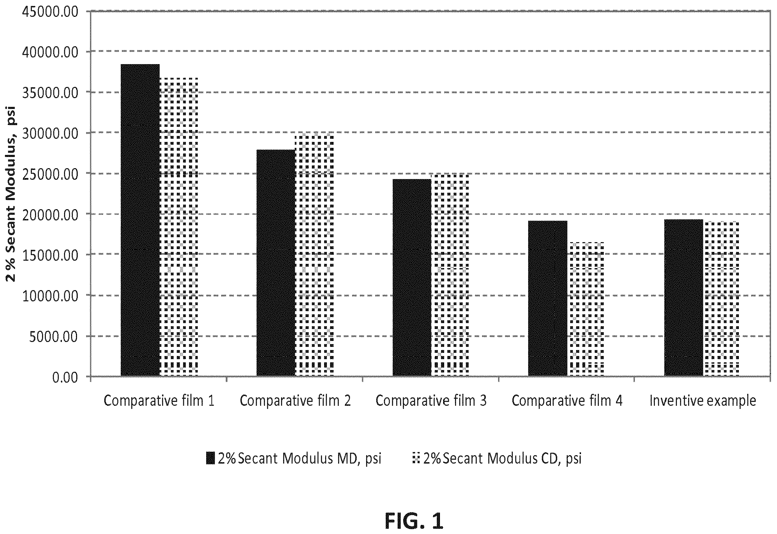

The 2% secant modulus (psi) was measured in the machine direction (MD) and cross direction (CD) for the inventive example and the comparative example films. The results are shown in Table 1. Referring to FIG. 1, the 2% secant modulus of the inventive example is lower than the 2% secant modulus of the comparative examples 1 and 2, which comprise greater amounts of polypropylene. In comparison to comparative examples 3 and 4, the 2% secant modulus of the inventive example has similar values showing that there is no significant adverse effect to the 2% secant modulus in the inventive example. Further, the 2% secant modulus of the inventive example achieved suitable levels, having values above a desired level of 110316 kPa (16,000 psi).

Stress at Break Results

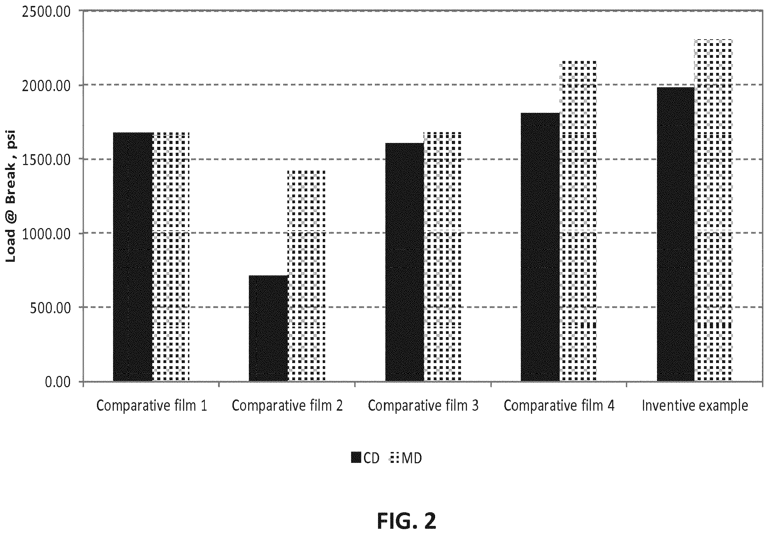

The stress or load at break (psi) was measured in the machine direction (MD) and cross direction (CD) for the inventive example and the comparative example films. The results are shown in Table 1. Referring to FIG. 2, the inventive example has a higher stress at break, which can indicate increased film strength in comparison to the comparative examples.

Spencer Dart Impact Strength Results

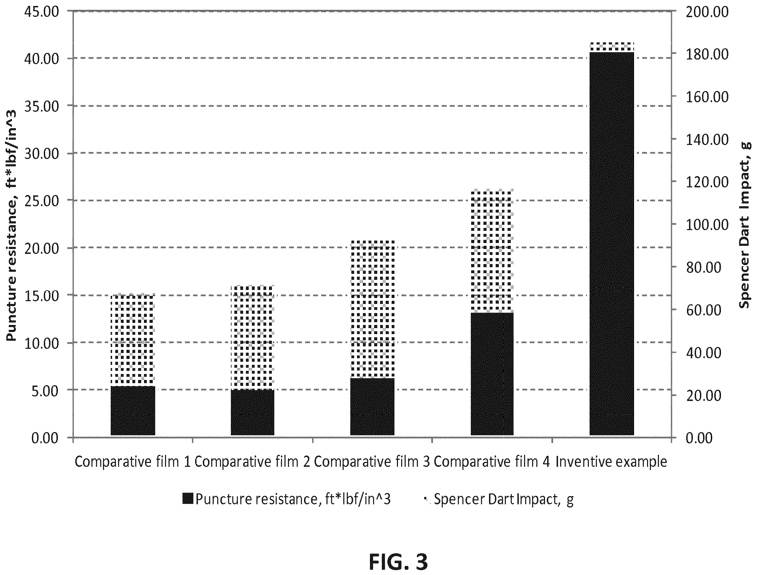

The spencer dart impact strength (g) was measured for the inventive example and the comparative example films. The results are shown in Table 1. Referring to FIG. 3, the inventive example has a higher dart impact strength, which can indicate increased biaxial film strength in comparison to the comparative examples.

Puncture Resistance Results

The puncture resistance (ft·lb$_f$/in$^3$) was measured for the inventive example and the comparative example films. The results are shown in Table 1. Still referring to FIG. 3, the inventive example has a higher puncture resistance, which can also indicate increased biaxial film strength in comparison to the comparative examples.

Noise Results

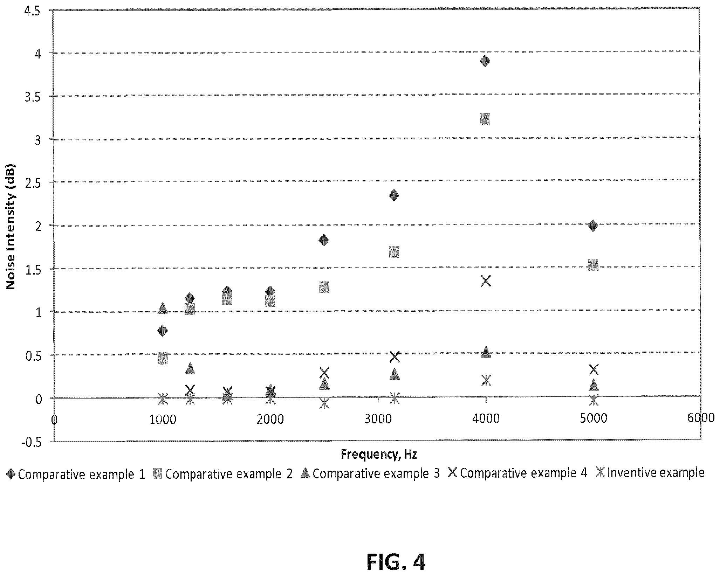

The noise (dB) was measured for the inventive example and the comparative example films between a frequency band of 20 Hz - 20,000 Hz. The results over the entire frequency band of 20 Hz - 20,000 Hz are shown in Table 1. Referring to FIG. 4, the noise between a frequency band of 1,000 - 5,000 Hz, which corresponds to the frequency band where a human ear is most sensitive to noise, is shown for the inventive example and the comparative examples. As depicted, the inventive example has much lower noise values than the comparative films.

Softness Results

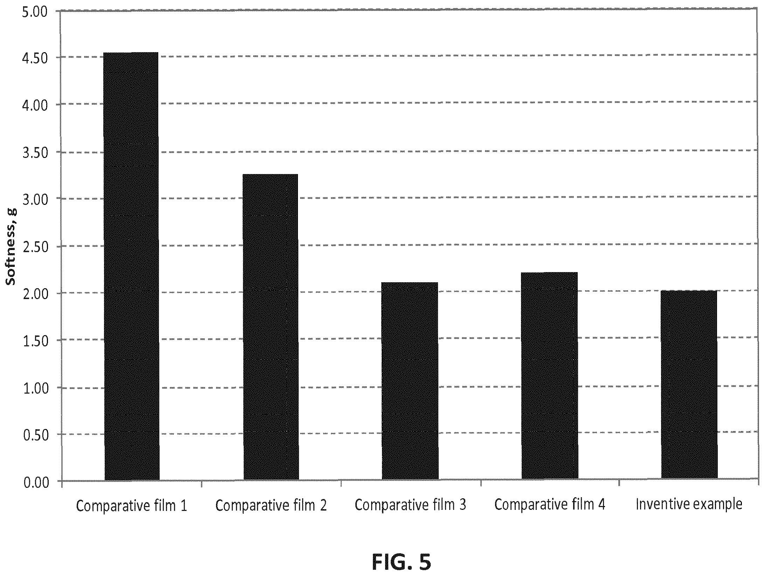

The softness (g) was measured for the inventive example and the comparative example films. The results are shown in Table 1. Referring to FIG. 5, the inventive example has a lower softness value, which can indicate a better softness result, than comparative examples 1 and 2, which use polypropylene. Also, the inventive example achieves suitable levels of softness as shown in comparison to comparative films 3 and 4. There is no significant adverse effect to softness in the inventive example.

Hydrostatic Pressure and Peel Force Results

The inventive example and the comparative example 4 films were ultrasonically bonded to a polypropylene nonwoven to form a laminate. The hydrostatic pressure present in the laminate and the peel force between the film and the nonwoven was measured for both the inventive example and comparative example 4. The table below shows that the inventive example showed a comparable suitable level of adhesion in comparison to comparative example 4 (100% polyethylene film), and the inventive example showed improved hydrostatic pressure performance over comparative example 4. The higher hydrostatic pressure of the inventive example can indicate a decrease in pinholes present in the laminate, whereas the lower hydrostatic pressure of the comparative example can indicate an increase in pinholes present in the laminate.

|  | Inventive Example | Comparative Example 4 |
| --- | --- | --- |
| Hydrostatic pressure [kPa (mbar)] | >7 (>70) | <2 (<20) |
| Peel Force (N) | 1.2 | 1 |

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

The citation of any document is not an admission that it is prior art with respect to any invention disclosed or claimed herein or that it alone, or in any combination with any other reference or references, teaches, suggests or discloses any such invention. Further, to the extent that any meaning or definition of a term in this document conflicts with any meaning or definition of the same term in a document referenced, the meaning or definition assigned to that term in this document shall govern.

**Claims**

1. A multilayer film comprising:

    a core layer; and
    two skin layers;
    wherein the core layer is positioned between the two skin layers;
    wherein the core layer comprises a polyethylene polymer blend, the polyethylene polymer blend comprising at least 40%, by weight of the polyethylene polymer blend, of an ethylene-based polymer having a density of 0.900-0.935 g/cc and a melt index of 0.7-6 g/10 min, wherein the polyethylene polymer blend has an overall density of about 0.910-0.945 g/cc and a melt index of about 0.7-6 g/ 10 min; and
    wherein each skin layer independently comprises a propylene-based polymer;

    wherein the propylene-based polymer:

    (a) is a propylene homopolymer; or
    (b) comprises a polypropylene polymer blend , wherein the polypropylene polymer blend comprises from 10 wt% to 30 wt% of a low density polyethylene and greater than 50 wt.% of a propylene-based polymer; and
    where the skin layer is not a non-woven material;
    wherein density is determined according to ASTM D-792; and
    wherein melt index is determined according to ASTM D1238 at 190 °C, 2.16 kg.

2. The film of claim 1, wherein the polypropylene polymer blend comprises at least about 60%, by weight of the polypropylene polymer blend, of the propylene-based polymer.

3. The film of claim 2, wherein the propylene homopolymer is isotactic, atactic or syndiotactic.

4. The film of claim 1, wherein the polyethylene polymer blend further comprises a low density polyethylene having a density of about 0.915-0.930 g/cc and a melt index of about 1-15 g/10 min.

5. The film of claim 1, wherein the polyethylene polymer blend comprises less than 30%, by weight of the polyethylene polymer blend, of the low density polyethylene.

6. The film of claim 1, wherein the polyethylene polymer blend further comprises a medium or high density polyethylene having a density of about 0.930-0.965 g/cc and a melt index of about 1-10 g/10 min.

7. The film of claim 1, wherein the polyethylene polymer blend comprises 15% to 30%, by weight of the polyethylene polymer blend, of the medium or high density polyethylene.

8. The film of claim 1, wherein the core layer comprises from about 50% to about 80% of the overall film thickness.

9. The film of claim 1, wherein the two skin layers have an equal thickness.

10. The film of claim 1, wherein the film exhibits at least one of the following properties:

    a spencer dart impact strength of greater than 140 g;
    a 2% secant modulus of greater than about 110316 kPa (16,000 psi) in the machine direction and greater than 110316 kPa (16,000 psi) in the cross direction;
    a stress at break in the cross-direction of greater than about 11721 kPa (1,700 psi), and in the machine direction of greater than about 13790 kPa (2,000 psi); or
    a puncture resistance greater than about 1.24 $J/cm^3$ (15 $ft \cdot lb_f/in^3$);
    wherein spencer dart impact strength is determined according to ASTM D3420, Procedure B;
    wherein 2% secant modulus is measured according to ASTM 882;.

11. The film of claim 1, wherein the film exhibits at least one of the following properties:
    a noise value of less than 0.5 dB between a frequency band of 1,000 Hz and 5,000 Hz.

12. The film of claim 1, wherein the film has a basis weight of between about 10-20 gsm.

13. An ultrasonically bonded laminate comprising:

    a multilayer film according to claim 1; and
    a nonwoven substrate at least partially ultrasonically bonded to the multilayer film, and wherein the laminate exhibits at least one of the following properties:

        a peel force value of greater than about 1.2 N; or
        a hydrostatic pressure above 7 kPa (70 mbar);
        wherein peel force is measured as specified in the description; and
        wherein hydrostatic pressure is determined according to ISO 1420.

14. A multilayer film comprising:

    a core layer; wherein the core layer comprises a polyethylene polymer blend, the polyethylene polymer blend comprising at least 40%, by weight of the polyethylene polymer blend, of an ethylene-based polymer having a density of 0.900-0.935 g/cc and a melt index of 0.7-6 g/10 min, wherein the polyethylene polymer blend has an overall density of about 0.910-0.945 g/cc and a melt index of about 0.7-6 g/ 10 min;
    a first layer that contact the core layer; where the first layer comprises a propylene-based polymer; where the first layer is not a non-woven material;
    wherein the propylene-based polymer:

        (a) is a propylene homopolymer; or
        (b) comprises a polypropylene polymer blend, wherein the polypropylene polymer blend comprises from 10 wt% to 30 wt% of a low density polyethylene and greater than 50 wt.% of a propylene-based polymer, by weight of the polypropylene polymer blend; and

    a nonwoven substrate at least partially ultrasonically bonded to the first layer on a surface that is opposed to a surface that contacts the core layer;
    wherein density is measured according to ASTM D-792; and

wherein melt index is measured according to ASTM D1238 at 190 °C, 2.16 kg.

**Patentansprüche**

1. Eine Mehrschichtfolie, die Folgendes beinhaltet:

   eine Kernschicht; und
   zwei Randschichten;
   wobei die Kernschicht zwischen den zwei Randschichten positioniert ist;
   wobei die Kernschicht eine Mischung aus Polyethylen und Polymer beinhaltet, wobei die Mischung aus Polyethylen und Polymer mindestens 40 %, nach Gewicht der Mischung aus Polyethylen und Polymer, eines Polymers auf Ethylenbasis mit einer Dichte von 0,900-0,935 g/cm$^3$ und einem Schmelzindex von 0,7-6 g/10 min beinhaltet,
   wobei die Mischung aus Polyethylen und Polymer eine Gesamtdichte von etwa 0,910-0,945 g/cm$^3$ und einen Schmelzindex von etwa 0,7-6 g/10 min beinhaltet; und
   wobei jede Randschicht unabhängig ein Polymer auf Propylenbasis beinhaltet;
   wobei das Polymer auf Propylenbasis:

      (a) ein Propylen-Homopolymer ist; oder
      (b) eine Mischung aus Polypropylen und Polymer beinhaltet, wobei die Mischung aus Polypropylen und Polymer von 10 Gew.-% bis 30 Gew.-% eines Polyethylens niederer Dichte und mehr als 50 Gew.-% eines Polymers auf Propylenbasis beinhaltet; und

   wobei die Randschicht kein Vliesstoff ist;
   wobei Dichte gemäß ASTM D-792 bestimmt wird; und
   wobei der Schmelzindex gemäß ASTM D1238 bei 190 °C, 2,16 kg, bestimmt wird.

2. Folie gemäß Anspruch 1, wobei die Mischung aus Polypropylen und Polymer mindestens etwa 60 %, nach Gewicht der Mischung aus Polypropylen und Polymer, des Polymers auf Propylenbasis beinhaltet.

3. Folie gemäß Anspruch 2, wobei das Propylen-Homopolymer isotaktisch, ataktisch oder syndiotaktisch ist.

4. Folie gemäß Anspruch 1, wobei die Mischung aus Polyethylen und Polymer ferner ein Polyethylen niederer Dichte mit einer Dichte von etwa 0,915-0,930 g/cm$^3$ und einem Schmelzindex von etwa 1-15 g/10 min beinhaltet.

5. Folie gemäß Anspruch 1, wobei die Mischung aus Polyethylen und Polymer weniger als 30 %, nach Gewicht der Mischung aus Polyethylen und Polymer, des Polyethylens niederer Dichte beinhaltet.

6. Folie gemäß Anspruch 1, wobei die Mischung aus Polyethylen und Polymer ferner ein Polyethylen mittlerer oder hoher Dichte mit einer Dichte von etwa 0,930-0,965 g/cm$^3$ und einem Schmelzindex von etwa 1-10 g/10 min beinhaltet.

7. Folie gemäß Anspruch 1, wobei die Mischung aus Polyethylen und Polymer 15 % bis 30 %, nach Gewicht der Mischung aus Polyethylen und Polymer, des Polyethylens mittlerer oder hoher Dichte beinhaltet.

8. Folie gemäß Anspruch 1, wobei die Kernschicht von etwa 50 % bis etwa 80 % der Gesamtfoliendicke beinhaltet.

9. Folie gemäß Anspruch 1, wobei die zwei Randschichten eine gleiche Dicke aufweisen.

10. Folie gemäß Anspruch 1, wobei die Folie mindestens eine der folgenden Eigenschaften vorweist:

    eine Spencer-Schlagfestigkeit von größer als 140 g;
    einen 2 %-Sekantenmodul von größer als etwa 110316 kPa (16,000 psi) in der Laufrichtung und größer als 110316 kPa (16,000 psi) in der Querrichtung;
    eine Bruchspannung in der Querrichtung von größer als etwa 11721 kPa (1700 psi) und
    in der Laufrichtung von größer als etwa 13790 kPa (2000 psi); oder
    eine Durchstichfestigkeit von größer als etwa 1,24 J/cm$^3$ (15 ft·lb$_f$/in$^3$);

wobei die Spencer-Schlagfestigkeit gemäß ASTM D3420, Prozedur B, bestimmt wird;
wobei der 2 %-Sekantenmodul gemäß ASTM 882 gemessen wird.

**11.** Folie gemäß Anspruch 1, wobei die Folie mindestens eine der folgenden Eigenschaften vorweist:

einen Geräuschwert von weniger als 0,5 dB zwischen einem Frequenzband von 1000 Hz und 5000 Hz.

**12.** Folie gemäß Anspruch 1, wobei die Folie ein Riesgewicht von zwischen etwa 10-20 gsm aufweist.

**13.** Ein mit Ultraschall gebundenes Laminat, das Folgendes beinhaltet:

eine Mehrschichtfolie gemäß Anspruch 1; und
ein Vliesstoffsubstrat, das zumindest teilweise mit Ultraschall an die Mehrschichtfolie gebunden ist, und wobei das Laminat mindestens eine der folgenden Eigenschaften vorweist:

einen Abziehfestigkeitswert von größer als etwa 1,2 N; oder
einen hydrostatischen Druck über 7 kPa (70 mbar);
wobei die Abziehfestigkeit wie in der Beschreibung spezifiziert gemessen wird; und
wobei der hydrostatische Druck gemäß ISO 1420 bestimmt wird.

**14.** Eine Mehrschichtfolie, die Folgendes beinhaltet:

eine Kernschicht; wobei die Kernschicht eine Mischung aus Polyethylen und Polymer beinhaltet, wobei die Mischung aus Polyethylen und Polymer mindestens 40 %, nach Gewicht der Mischung aus Polyethylen und Polymer, eines Polymers auf Ethylenbasis beinhaltet, das eine Dichte von 0,900-0,935 g/cm$^3$ und einen Schmelzindex von 0,7-6 g/10 min aufweist, wobei die Mischung aus Polyethylen und Polymer eine Gesamtdichte von etwa 0,910-0,945 g/cm$^3$ und einen Schmelzindex von etwa 0,7-6 g/10 min aufweist;
eine erste Schicht, die die Kernschicht kontaktiert; wobei die erste Schicht ein Polymer auf Propylenbasis beinhaltet; wobei die erste Schicht kein Vliesstoff ist;
wobei das Polymer auf Propylenbasis:

(a) ein Propylen-Homopolymer ist; oder
(b) eine Mischung aus Polypropylen und Polymer beinhaltet, wobei die Mischung aus Polypropylen und Polymer von 10 Gew.-% bis 30 Gew.-% eines Polyethylens niederer Dichte und mehr als 50 Gew.-% eines Polymers auf Propylenbasis, nach Gewicht der Mischung aus Polypropylen und Polymer, beinhaltet; und

ein Vliesstoffsubstrat, das mindestens teilweise mit Ultraschall an die erste Schicht an einer Oberfläche gebunden ist, die gegenüber einer Oberfläche liegt, die die Kernschicht kontaktiert;
wobei die Dichte gemäß ASTM D-792 gemessen wird; und
wobei der Schmelzindex gemäß ASTM D1238 bei 190 °C, 2,16 kg, gemessen wird.

**Revendications**

**1.** Un film multicouche comprenant:

une couche centrale ; et
deux couches de peau ;
dans lequel la couche centrale est positionnée entre les deux couches de peau ;
dans lequel la couche centrale comprend un mélange homogène de polymères de polyéthylène, le mélange homogène de polymères de polyéthylène comprenant au moins 40 %, en poids du mélange homogène de polymères de polyéthylène, d'un polymère à base d'éthylène ayant une masse volumique de 0,900 à 0,935 g/cm$^3$ et un indice de fluidité à l'état fondu de 0,7 à 6 g/10 min, dans lequel le mélange homogène de polymères de polyéthylène a une masse volumique apparente d'environ 0,910 à 0,945 g/cm$^3$ et un indice de fluidité à l'état fondu d'environ 0,7 à 6 g/10 min ; et
dans lequel chaque couche de peau comprend indépendamment un polymère à base de propylène ;
dans lequel le polymère à base de propylène :

(a) est un homopolymère de propylène ; ou

(b) comprend un mélange homogène de polymères de polypropylène, le mélange homogène de polymères de polypropylène comprenant de 10 % en poids à 30 % en poids d'un polyéthylène basse densité et plus de 50 % en poids d'un polymère à base de propylène ; et

où la couche de peau n'est pas un matériau non tissé ;

dans lequel la masse volumique est déterminée selon l'ASTM D-792 ; et

dans lequel l'indice de fluidité à l'état fondu est déterminé selon l'ASTM D1238 à 190 °C, 2,16 kg.

2. Le film de la revendication 1, dans lequel le mélange homogène de polymères de polypropylène comprend au moins environ 60 %, en poids du mélange homogène de polymères de polypropylène, du polymère à base de propylène.

3. Le film de la revendication 2, dans lequel l'homopolymère de propylène est isotactique, atactique ou syndiotactique.

4. Le film de la revendication 1, dans lequel le mélange homogène de polymères de polyéthylène comprend en outre un polyéthylène basse densité ayant une masse volumique d'environ 0,915 à 0,930 g/cm$^3$ et un indice de fluidité à l'état fondu d'environ 1 à 15 g/10 min.

5. Le film de la revendication 1, dans lequel le mélange homogène de polymères de polyéthylène comprend moins de 30 %, en poids du mélange homogène de polymères de polyéthylène, du polyéthylène basse densité.

6. Le film de la revendication 1, dans lequel le mélange homogène de polymères de polyéthylène comprend en outre un polyéthylène moyenne ou haute densité ayant une masse volumique d'environ 0,930 à 0,965 g/cm$^3$ et un indice de fluidité à l'état fondu d'environ 1 à 10 g/10 min.

7. Le film de la revendication 1, dans lequel le mélange homogène de polymères de polyéthylène comprend de 15 % à 30 %, en poids du mélange homogène de polymères de polyéthylène, du polyéthylène moyenne ou haute densité.

8. Le film de la revendication 1, dans lequel la couche centrale constitue d'environ 50 % à environ 80 % de l'épaisseur de film totale.

9. Le film de la revendication 1, dans lequel les deux couches de peau ont une épaisseur égale.

10. Le film de la revendication 1, le film faisant preuve d'au moins une des propriétés suivantes :

une résistance à l'impact d'un projectile spencer de plus de 140 g ;

un module sécant à 2 % de plus d'environ 110 316 kPa (16 000 psi) dans le sens machine et de plus de 110 316 kPa (16 000 psi) dans le sens travers ;

une contrainte à la rupture dans le sens travers de plus d'environ 11 721 kPa (1 700 psi), et dans le sens machine de plus d'environ 13 790 kPa (2 000 psi) ; ou

une résistance à la perforation de plus d'environ 1,24 J/cm$^3$ (15 pi·lb$_f$/po$^3$) ;

dans lequel la résistance à l'impact d'un projectile spencer est déterminée selon l'ASTM D3420, Procédure B ;

dans lequel le module sécant à 2 % est mesuré selon l'ASTM 882.

11. Le film de la revendication 1, le film faisant preuve d'au moins une des propriétés suivantes :

une valeur de bruit de moins de 0,5 dB entre une bande de fréquences de 1 000 Hz et 5 000 Hz.

12. Le film de la revendication 1, le film ayant un poids de base compris entre environ 10 et 20g/m$^2$.

13. Un stratifié lié par ultrasons comprenant :

un film multicouche selon la revendication 1 ; et

un substrat non tissé lié par ultrasons au moins partiellement au film multicouche, et le stratifié faisant preuve d'au moins une des propriétés suivantes :

une valeur de force de décollement de plus d'environ 1,2 N ; ou

une pression hydrostatique au-dessus de 7 kPa (70 mbar) ;

dans lequel la force de décollement est mesurée telle que spécifiée dans la description ; et
dans lequel la pression hydrostatique est déterminée selon l'ISO 1420.

14. Un film multicouche comprenant :

une couche centrale ; la couche centrale comprenant un mélange homogène de polymères de polyéthylène, le mélange homogène de polymères de polyéthylène comprenant au moins 40 %, en poids du mélange homogène de polymères de polyéthylène, d'un polymère à base d'éthylène ayant une masse volumique de 0,900 à 0,935 g/cm$^3$ et un indice de fluidité à l'état fondu de 0,7 à 6 g/10 min, dans lequel le mélange homogène de polymères de polyéthylène a une masse volumique apparente d'environ 0,910 à 0,945 g/cm$^3$ et un indice de fluidité à l'état fondu d'environ 0,7 à 6 g/10 min;
une première couche qui est en contact avec la couche centrale ; où la première couche comprend un polymère à base de propylène ; où la première couche n'est pas un matériau non tissé ;
dans lequel le polymère à base de propylène :

(a) est un homopolymère de propylène ; ou
(b) comprend un mélange homogène de polymères de polypropylène, le mélange homogène de polymères de polypropylène comprenant de 10 % en poids à 30 % en poids d'un polyéthylène basse densité et plus de 50 % en poids d'un polymère à base de propylène, en poids du mélange homogène de polymères de polypropylène ; et

un substrat non tissé lié par ultrasons au moins partiellement à la première couche sur une surface qui est opposée à une surface qui est en contact avec la couche centrale ;
dans lequel la masse volumique est mesurée selon l'ASTM D-792 ; et
dans lequel l'indice de fluidité à l'état fondu est mesuré selon l'ASTM D1238 à 190 °C, 2,16 kg.

**FIG. 1**

■ 2% Secant Modulus MD, psi    ⠿ 2% Secant Modulus CD, psi

Comparative film 1    Comparative film 2    Comparative film 3    Comparative film 4    Inventive example

2% Secant Modulus, psi

45000.00
40000.00
35000.00
30000.00
25000.00
20000.00
15000.00
10000.00
5000.00
0.00

**FIG. 2**

**FIG. 3**

FIG. 4

FIG. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2003106560 A1
- US 5272236 A
- US 5278272 A
- US 5582923 A
- US 5733155 A
- US 3645992 A
- US 4076698 A
- US 3914342 A
- US 5854045 A
- WO 2005111291 A1
- US 4599392 A
- WO 2005023912 A
- WO 2007081548 A
- WO 1998004397 A

**Non-patent literature cited in the description**

- **RANDALL.** *Rev. Macromol. Chem. Phys.,* vol. C29 (2,3), 285-297
- **ZIMM, B. H. ; STOCKMAYER, W. H.** *J. Chem. Phys.,* 1949, vol. 17, 1301
- **RUDIN A.** Modern Methods of Polymer Characterization. John Wiley & Sons, 1991, 103-112